# EUROPEAN PATENT APPLICATION

(11) **EP 4 714 468 A1**
(43) Date of publication of application: **25.03.2026**
(21) Application number: 24807539.2
(22) Date of filing: 14.05.2024
(51) Int. Cl.: A61K 45/00, A61P 17/14

(54) **METHOD FOR ADMINISTERING MODULATOR TARGETING S1PR1 AND S1PR4**

(30) Priority: 15.05.2023 KR 20230062582
(71) Applicant: Nextgen Bioscience Co., Ltd., Seongnam-si, Gyeonggi-do 13487 (KR)
(72) Inventor: LEE, Bongyong, Seoul 06625 (KR); PARK, Yanghae, Seoul 04421 (KR); KIM, Hee Sun, Seoul 06224 (KR)
(74) Representative: dompatent
(86) International application number: PCT/KR2024/006583
(87) International publication number: WO 2024/237668

(57) **Abstract**

The present invention relates to a method for administering a modulator targeting S1PR1 and S1PR4. The present invention can be effectively used in the treatment of various diseases correlated with the absolute lymphocyte count, such as alopecia, inflammatory bowel disease, interstitial fibrosis, coronary atrophy, and focal segmental glomerulosclerosis, by providing the optimal dosage and frequency of administration of a modulator (NXC736) targeting S1PR1 and S1PR4, which is capable of exhibiting excellent safety when administered to the human body and inhibiting the absolute lymphocyte count by 50% or more.

## Description

### TECHNICAL FIELD

The present invention relates to a method for administering a modulator targeting S1PR1 and S1PR4, and more specifically to a method for administering a modulator targeting S1PR1 and S1PR4 that can effectively suppress the number of lymphocytes in a human body and effectively treat diseases mediated by lymphocytes.

### BACKGROUND ART

NXC736 has a mechanism of action that binds to the Sphingosine-1 Phosphate (S1P) receptor, thereby inducing a functional antagonist effect at the receptor, and exhibiting immunomodulatory and anti fibrotic effects. In particular, NXC736 is a compound that selectively binds to S1P1 and S1P4, compared to Fingolimod, which non-selectively binds to S1P1, 3, 4 and 5 subtypes. Fingolimod, which was first approved as a functional antagonist acting on the S1P receptor, had adverse cardiovascular and respiratory side effects due to S1P3 binding, and thereafter, the need for the development of drugs that selectively act on S1P has arisen. Currently, a total of four substances, including Fingolimod, have received global product approval for indications of multiple sclerosis and ulcerative colitis, but NXC736 is the only substance that selectively binds to S1P1 and S1P4.

NXC736 has been demonstrated to effectively treat various lymphocyte-mediated diseases, such as alopecia areata, inflammatory bowel disease, interstitial fibrosis and tubular atrophy, and focal segmental glomerulosclerosis, through animal experiments (Korean Patent No. 10-2541577, Korean Patent No. 10-2541578, Korean Patent No. 10-2563719, Korean Patent No. 10-2541579).

However, these experiments only confirmed that a single dose of NXC736 has a therapeutic effect on various lymphocyte-mediated diseases in animals, and there was no disclosure or indication of an appropriate administration dose or administration method that would show a therapeutic effect on lymphocyte-mediated diseases in humans.

Accordingly, there has been a demand for the establishment of a safer and more effective dosage and administration cycle method for the treatment of diseases mediated by lymphocytes.

### DISCLOSURE

### TECHNICAL PROBLEM

An object of the present invention is to provide a safe and effective dosage and administration cycle of a modulator targeting S1PR1 and S1PR4 in the prevention or treatment of lymphocyte-mediated diseases.

### TECHNICAL SOLUTION

In order to achieve the above object, the present invention provides a pharmaceutical composition for preventing or treating a lymphocyte-mediated disease, including a compound represented by Chemical Formula 1 or a pharmaceutically acceptable salt, hydrate or solvate thereof as an active ingredient, wherein the active ingredient is administered to a subject in need thereof at a dose equivalent to 40 to 80 mg of the compound represented by Chemical Formula 1: 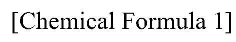 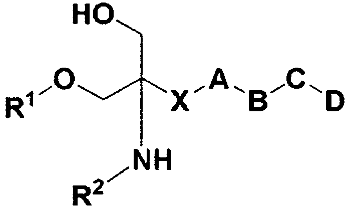 wherein
R¹ is hydrogen;
R² is hydrogen or an acetyl group;
X is a single bond, C₂ alkylene or C₂ alkenylene;
A is a five-membered heteroarylene ring including three N atoms;
B is a C₂₋₁₁ straight-chain or branched-chain alkylene;
C is a single bond or phenylene; and
D is selected from the group consisting of hydrogen, phenyl and C₁₋₆ alkyl.

In the present invention, the compound represented by Chemical Formula 1 may be a compound represented by Chemical Formula 2:

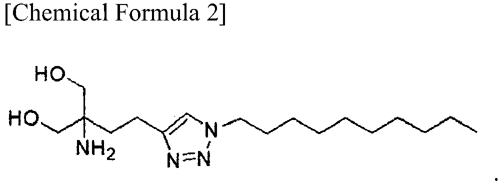

In the present invention, the active ingredient may be administered at a dose of 50 to 70 mg.

In the present invention, the active ingredient may be administered at a dose of 60 mg.

In the present invention, the pharmaceutical composition may be administered once a day or twice a day.

In the present invention, the pharmaceutical composition may be administered for at least 14 days.

In the present invention, the pharmaceutical composition may be administered orally.

In the present invention, the pharmaceutical composition may be formulated as a capsule or tablet that is suitable for oral administration.

In the present invention, the pharmaceutical composition may further include a pharmaceutically acceptable carrier.

In the present invention, the pharmaceutical composition may suppress an absolute lymphocyte count to 50% or less.

In the present invention, the lymphocyte-mediated disease may be alopecia areata, inflammatory bowel disease, interstitial fibrosis and tubular atrophy or focal segmental glomerulosclerosis.

### ADVANTAGEOUS EFFECTS

The present invention provides an optimal administration dosage and cycle of a modulator (NXC736) targeting S1PR1 and S1PR4, which exhibits excellent safety when administered to humans and can suppress an absolute lymphocyte count by 50% or more, and it can be effectively used in the treatment of various diseases that are correlated with an absolute lymphocyte count, such as alopecia areata, inflammatory bowel disease, interstitial fibrosis and tubular atrophy and focal segmental glomerulosclerosis.

### DESCRIPTION OF DRAWINGS

FIG. 1 shows the mean NXC736 concentration-time profile after a single administration of NXC736.
FIG. 2 shows the mean NXC736 concentration-time profile after repeated administrations of NXC736.
FIG. 3 shows the results of comparing the PK parameters (C_{max,ss} and AUC_{tau,ss}) of NXC736 after repeated administrations of NXC736 by dosage group.
FIG. 4 shows the mean percentage changes in absolute lymphocyte counts from the baseline time profile after a single administration of NXC736.
FIG. 5 shows the mean percentage changes in absolute lymphocyte counts from the baseline time profile after repeated administrations of NXC736.
FIG. 6 shows the results of confirming changes in absolute lymphocyte counts over time compared to the initial absolute lymphocyte count (t=0) after a single (QD) administration of NXC736 at a dose of 20 mg/kg to healthy rats or two repeated (BID) administrations at a dose of 5 mg/kg or 10 mg/kg at 12-hour intervals.
FIG. 7 shows the results of checking the alopecia areata lesion area (AA lesion area) at two-week intervals after orally administering NXC736 at a dose of 3 mg/kg or 10 mg/kg twice daily (BID) for 12 weeks to alopecia areata model mice.
FIG. 8 shows the results comparing the pharmacodynamic parameters (Δ_{max,ss}) of NXC736 after single or repeated administrations of NXC736.
FIG. 9 shows the average maximum percentage change in absolute lymphocyte count compared to baseline.

### MODES OF THE INVENTION

Unless otherwise defined, all technical and scientific terms used herein have the same meaning as commonly understood by one of ordinary skill in the art to which this invention pertains. In case of conflict, the present application, including its definitions, will control. Unless the context otherwise requires, singular terms include plurals and plural terms include singulars. All publications, patents and other references mentioned herein are incorporated by reference in their entirety for all purposes as if each individual publication or patent application was specifically and individually indicated to be incorporated by reference.

### Definitions

As used herein, the term "about" is used to mean approximately, nearly, approximately or to a degree. When the term "about" is used in conjunction with a numerical range, the term modifies the range by extending the upper and lower boundaries of the stated numerical values. Typically, the term "about" is used to modify a numerical value above or below a stated value by a variation of 10 percent above or below (higher or lower) the stated numerical value.

In all instances where aspects are described herein with the expression "including", it should be understood that other similar aspects described in terms of "consisting of" and/or "consisting essentially of' are also provided.

In addition, it should also be understood that in all cases where the present specification is described in the form of a pharmaceutical composition as an embodiment, this is also provided in other similar forms described in the form of a pharmaceutical use form, a Swiss-type pharmaceutical use form and/or a therapeutic method form for using the same.

As used herein, "an effective dose" refers to an amount effective and/or for a period of time necessary to achieve the desired therapeutic outcome. The therapeutic outcome may be, for example, relief of symptoms, prolonged survival and the like. The therapeutic outcome need not be a "cure."

As used herein, terms such as "treating" or "treatment" or "to treat" or "alleviating" or "to alleviate" refer to therapeutic means for curing, delaying, reducing symptoms and/or halting the progression of a diagnosed pathological condition or disease. Accordingly, subjects in need of treatment include those who have already been diagnosed with a disease or are suspected of having a disease.

A "subject" or "individual" or "animal" or "patient" or "mammal" means any subject for which diagnosis, prognosis or treatment is desired, particularly a mammalian subject. Mammalian subjects include, but are not limited to, humans, livestock, farm animals, zoo animals, sport animals, pets, such as dogs, cats, guinea pigs, rabbits, rats, mice, horses, cows, dairy cows; primates, such as apes, monkeys, orangutans and chimpanzees; canids, such as dogs and wolves; felines, such as cats, lions and tigers; equines, such as horses, donkeys and zebras; bears, food animals, such as cattle, pigs and sheep; ungulates, such as deer and giraffes; rodents, such as mice, rats, hamsters and guinea pigs; and the like. In certain embodiments, the mammal is a human subject.

As used herein, a functional antagonist refers to a moiety that competitively binds to a receptor at the same site as an agonist (e.g., an endogenous ligand), but does not activate an intracellular response initiated by the active form of the receptor, thereby inhibiting an intracellular response by an agonist or partial agonist. An antagonist does not decrease the baseline intracellular response in the absence of an agonist or partial agonist.

As used herein, an agonist refers to a moiety that interacts with and activates a G-protein-coupled receptor, such as S1PR1 and S1PR4, and thereby initiates a physiological or pharmacological response characteristic of that receptor. For example, an agonist activates an intracellular response upon binding to a receptor, or enhances GTP binding to a membrane. In certain embodiments, an agonist of the invention is an S1PR1 and S1PR4 agonist that can facilitate sustained S1PR1 and S1PR4 internalization.

### Therapeutic substance (active ingredient)

The present invention provides a compound represented by Chemical Formula 1 below, or a pharmaceutically acceptable salt, hydrate or solvate thereof, as an active ingredient: 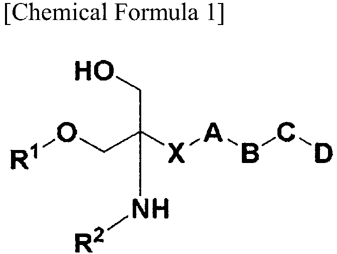 wherein
R¹ is hydrogen;
R² is hydrogen or an acetyl group;
X is a single bond, C₂ alkylene or C₂ alkenylene;
A is a five-membered heteroarylene ring including three N atoms;
B is a C₂₋₁₁ straight-chain or branched-chain alkylene;
C is a single bond or phenylene; and
D is selected from the group consisting of hydrogen, phenyl and C₁₋₆ alkyl.

The compound represented by Chemical Formula 1 may be any one compound selected from the following chemical group:
(1) 2-Amino-2-(2-(1-decyl-1H-1,2,3-triazol-4-yl)ethyl)propane-1,3-diol;
(2) 2-Amino-2-(2-(1-octyl-1H-1,2,3-triazol-4-yl)ethyl)propane-1,3-diol;
(3) 2-Amino-2-(2-(1-(4-hexylphenethyl)-1H-1,2,3-triazol-4-yl)ethyl)propane-1,3-diol;
(4) 2-Amino-2-(1-dodecyl-1H-1,2,3-triazol-4-yl)propane-1,3-diol;
(5) (E)-2-Amino-2-(1-decyl-1H-1,2,3-triazol-4-yl)vinyl-1,3-diol;
(6) 2-Amino-2-(2-(1-(8-phenyloctyl)-1H-1,2,3-triazolebutyl-4-yl)ethyl)propane-1,3-diol;
(7) N-(2-(1-dodecyl-1H-1,2,3-triazol-4-yl)-1,3-dihydrooxypropan-2-yl)acetamide;
(8) N-(4-(1-decyl-1H-1,2,3-triazol-4-yl)-1-hydroxy-2-(hydroxymethyl)butan-2-yl)acetamide; and
(9) N-(4-(1-(4-hexylphenethyl)-1H-1,2,3-triazol-4-yl)-1-hydroxy-2-(hydroxymethyl)butan-2-yl)acetamide.

The compound represented by Chemical Formula 1 may be a compound represented by Chemical Formula 2.

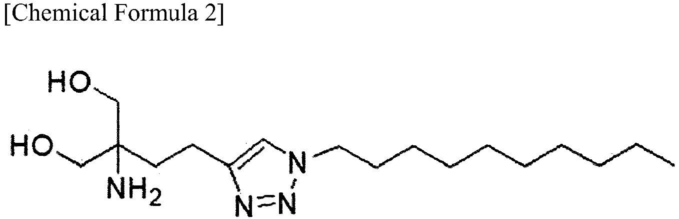

In the present specification, the compound represented by Chemical Formula 2 may be used interchangeably with the term 'NXC736'.

In the present specification, any formulation of the examples including the compound represented by Chemical Formula 2 as an active ingredient may be used interchangeably with a 'clinical trial drug.'

The compound according to the present invention may optionally be present as a pharmaceutically acceptable salt, for example a pharmaceutically acceptable acid addition salt prepared from a pharmaceutically acceptable non-toxic acid, for example inorganic acids and organic acids. Representative acids include, but are not limited to, acetic acid, benzenesulfonic acid, benzoic acid, camphorsulfonic acid, citric acid, ethenesulfonic acid, dichloroacetic acid, formic acid, fumaric acid, gluconic acid, glutamic acid, hippuric acid, hydrobromic acid, hydrochloric acid, isethionic acid, lactic acid, maleic acid, malic acid, mandelic acid, methanesulfonic acid, mucinic acid, nitric acid, oxalic acid, pamoic acid, pantothenic acid, phosphoric acid, succinic acid, sulfuric acid, tartaric acid, oxalic acid, p-toluenesulfonic acid and the like, and for example, those pharmaceutically acceptable salts of which are listed in the literature [Berge et al., Journal of Pharmaceutical Sciences, 66:1-19 (1977)], which is incorporated herein by reference in its entirety.

As used herein, "a hydrate" means a compound of the present invention or a salt thereof, which additionally includes a stoichiometric or non-stoichiometric amount of water bound by non-covalent intermolecular forces.

As used herein, "a solvate" means a compound of the present invention or a salt thereof, which further includes a stoichiometric or non-stoichiometric amount of a solvent bound by non-covalent intermolecular forces. Preferred solvents are volatile, non-toxic and/or traceable to humans.

### Treatment method (dosage and administration regimen)

In the present invention, the administration dosage that ensures the safety of NXC736 was confirmed in a clinical trial conducted on healthy adults, and an administration method that is capable of effectively reducing an absolute lymphocyte count in the blood by administering NXC736 was confirmed, thereby providing an effective administration method of NXC736.

Particularly, in the present invention, when an administration method that is capable of effectively reducing an absolute lymphocyte count in the blood by administering NXC736 to a healthy animal was applied to an animal model of alopecia areata, which is one of the lymphocyte-mediated diseases, it was demonstrated through animal experiments that the disease could actually be effectively treated.

Absolute lymphocyte count has been used as a biomarker for the onset or diagnosis of alopecia areata, inflammatory bowel disease, interstitial fibrosis and tubular atrophy, and focal segmental glomerulosclerosis (see J Clin Invest. 2007 Aug 1; 117(8): 2019-2027. doi: 10.1172/JC131942, Biomed Res Int. 2018; 2018: 4961753. doi: 10.1155/2018/4961753, J Clin Lab Anal. 2021 Jun; 35(6): e23774. doi: 10.1002/jcla.23774, BMJ Case Rep. 2015; 2015: bcr2015211765. doi: 10.1136/ber-2015-211765), and those skilled in the art use the suppression of an absolute lymphocyte count as a means of determining the therapeutic effect of the disease.

Accordingly, the present invention provides a pharmaceutical composition for preventing or treating a lymphocyte-mediated disease, including the therapeutic substance as an active ingredient, wherein the active ingredient is administered to a subject in need thereof at a dose equivalent to about 40 mg to about 80 mg of a compound represented by Chemical Formula 1 (in one embodiment, a compound represented by Chemical Formula 2).

For example, it provides a pharmaceutical composition, wherein the active ingredient is administered to a subject in need thereof at a dose equivalent to about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, about 70 mg, about 71 mg, about 72 mg, about 73 mg, about 74 mg, about 75 mg, about 76 mg, about 77 mg, 78 mg, about 79 mg or about 80 mg of the compound represented by Chemical Formula 1 (in one aspect, the compound represented by Chemical Formula 2).

In addition, the active ingredient may be administered to a subject in need thereof in a dosage equivalent to a dosage within any range of about 40 mg to about 80 mg of the compound represented by Chemical Formula 1 (in one aspect, the compound represented by Chemical Formula 2).

In one aspect, the active ingredient may be administered at a dose equivalent to about 50 mg to about 70 mg of the compound represented by Chemical Formula 1 (in one aspect, the compound represented by Chemical Formula 2).

In another aspect, the active ingredient may be administered at a dose equivalent to about 55 mg to about 65 mg of the compound represented by Chemical Formula 1 (in one aspect, the compound represented by Chemical Formula 2).

In still another aspect, the active ingredient may be administered at a dose equivalent to about 60 mg of the compound represented by Chemical Formula 1 (in one aspect, the compound represented by Chemical Formula 2).

In the present invention, the pharmaceutical composition may be administered once a day, twice a day, or three times a day.

In one aspect, the pharmaceutical composition may be administered twice a day.

The pharmaceutical composition has an advantage that the decrease in absolute lymphocyte count is maintained for a longer period of time when administered twice a day than when administered once a day.

In the present invention, the pharmaceutical composition may be administered at intervals of about 6 hours to about 24 hours.

For example, the pharmaceutical composition may be administered for about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours or about 24 hours.

In one aspect, the pharmaceutical composition may be administered at intervals of about 8 hours to about 16 hours.

In another aspect, the pharmaceutical composition may be administered at intervals of about 10 hours to about 14 hours.

In still another aspect, the pharmaceutical composition may be administered at intervals of about 11 hours to about 13 hours.

In still another aspect, the pharmaceutical composition may be administered at intervals of about 12 hours.

In the present invention, the pharmaceutical composition may be administered for at least 14 days.

For example, the pharmaceutical composition may be administered for at least about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, about 30 days, about 31 days, about 32 days, about 33 days, about 34 days, about 35 days, about 36 days, about 37 days, about 38 days, about 39 days, about 40 days, about 41 days, about 42 days, about 43 days, about 44 days, about 45 days, about 46 days, about 47 days, about 48 days, about 49 days or about 50 days.

In addition, the pharmaceutical composition may be administered for at least about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, or about 52 weeks or more.

In addition, the pharmaceutical composition may be administered for at least about 1 month, about 2 months, about 3 months, about 4 months, about 5 months, about 6 months, about 7 months, about 8 months, about 9 months, about 10 months, about 11 months or about 12 months or more.

In the present invention, the pharmaceutical composition may be administered orally or parenterally.

In one embodiment, the pharmaceutical composition may be administered orally.

In the present invention, the pharmaceutical composition may be formulated as a capsule or tablet that is suitable for oral administration.

In the present invention, the pharmaceutical composition may further include a pharmaceutically acceptable carrier.

In one embodiment, the active ingredient may be administered as an unprocessed or pure chemical substance, for example, as a powder in a capsule formulation.

In another aspect, the active ingredient may be formulated and administered as a pharmaceutical composition further including a pharmaceutically acceptable carrier.

Pharmaceutical compositions may be prepared by any suitable method, typically by homogeneously mixing the active compound(s) with a liquid or finely divided solid carrier, or both, in the required proportions and then, if desired, shaping the resulting mixture into the desired shape.

In pharmaceutical compositions, conventional excipients, such as binders, fillers, acceptable wetting agents, tableting lubricants and disintegrating agents, may be used in tablets and capsules for oral administration. Liquid preparations for oral administration may be in the form of solutions, emulsions, aqueous or oily suspensions and syrups. Alternatively, the oral preparations may be in the form of dry powders that may be reconstituted with water or another suitable liquid vehicle before use. Additional additives, such as suspending or emulsifying agents, non-aqueous vehicles (including edible oils), preservatives and flavoring and coloring agents may be added to the liquid preparations. Parenteral dosage forms may be prepared by dissolving a compound of the present invention in a suitable liquid vehicle, sterile filtering the solution, and then filling and sealing it in a suitable vial or ampoule. These are just a few examples of many suitable methods well known in the art for the preparation of dosage forms.

The compound of the present invention may be formulated into a pharmaceutical composition by using techniques well known to those skilled in the art. Suitable pharmaceutically acceptable carriers other than those mentioned herein are known in the art; see, for example, Remington, The Science and Practice of Pharmacy, 20th Edition, 2000, Lippincott Williams & Wilkins, (Editors: Gennaro et al.).

Pharmaceutical formulations include those suitable for oral, rectal, nasal, topical (e.g., buccal and sublingual), vaginal or parenteral (e.g., intramuscular, subcutaneous, and intravenous) administration, or for administration by inhalation, insufflation or transdermal patch. Transdermal patches dispense a drug at a controlled rate by presenting the drug to be absorbed in an effective manner with minimal degradation of the drug. Typically, a transdermal patch includes an impermeable backing layer, a single pressure-sensitive adhesive, and a removable protective layer having a release liner. Those skilled in the art will understand and appreciate appropriate techniques for fabricating an effective transdermal patch as desired, depending on the needs of a person skilled in the art.

Accordingly, the compound of the present invention may be incorporated into pharmaceutical preparations and unit dosage forms thereof together with conventional adjuvants, carriers or diluents, and may be used in such forms as solids, such as tablets or filled capsules, or liquids, such as solutions, suspensions, emulsions, elixirs, gels or filled capsules thereof (all for oral use); in the form of suppositories for rectal administration; or in the form of sterile injectable solutions for parenteral (including subcutaneous) use. Such pharmaceutical compositions and unit dosage forms thereof may include the conventional ingredients in the conventional proportions, with or without additional active compounds or ingredients, and such unit dosage forms may contain any suitable effective amount of the active ingredient corresponding to the intended daily dosage range for use.

In the case of oral administration, the pharmaceutical composition may be, for example, in the form of a tablet, capsule, suspension or liquid. The pharmaceutical composition is preferably prepared in the form of a dosage unit containing a specific amount of the active ingredient. Examples of such dosage units are capsules, tablets, powders, granules or suspensions containing conventional additives, such as lactose, mannitol, corn starch or potato starch; binders, such as crystalline cellulose, cellulose derivatives, acacia, corn starch or gelatin; disintegrants, such as corn starch, potato starch or sodium carboxymethylcellulose; and lubricants, such as talc or magnesium stearate. The active ingredient may also be administered by injection as a composition, and for example, saline, dextrose or water may be used as a suitable pharmaceutically acceptable carrier.

For preparing a pharmaceutical composition from the compound of the present invention, suitable pharmaceutically acceptable carriers may be solids, liquids or mixtures of both. Solid form preparations include powders, tablets, pills, capsules, cachets, suppositories and dispersible granules. The solid carrier may be one or more ingredients, which can also act as diluents, flavoring agents, solubilizers, lubricants, suspending agents, binders, preservatives, tablet disintegrating agents or encapsulating materials.

In powders, the carrier may be a finely divided solid that is present in a mixture with the finely divided active ingredient.

In the tablet, the active ingredient may be mixed with a carrier having the necessary binding capacity in a suitable ratio, and may be compressed into the desired shape and size.

Powders and tablets may contain various percentages of the active ingredient. A typical amount in a powder or tablet may be from 0.5 to about 99 percent of the active ingredient. However, those skilled in the art will recognize that amounts outside these ranges may be required. Suitable carriers for powders and tablets include magnesium carbonate, magnesium stearate, talc, sugar, lactose, pectin, dextrin, starch, gelatin, tragacanth, methylcellulose, sodium carboxymethylcellulose, low melting waxes, cocoa butter and the like. The term "formulation" is intended to include formulating the active ingredient together with an encapsulating material as a carrier, so as to provide a capsule in which the active ingredient is surrounded by and associated with the carrier, with or without the carrier. Similarly, cachets and lozenges are included. Tablets, powders, capsules, pills, cachets and lozenges may be used as solid forms suitable for oral administration.

Liquid form preparations include solutions, suspensions and emulsions, and for example, water or water-propylene glycol solutions. For example, parenteral injectable liquid preparations may be formulated as solutions in aqueous polyethylene glycol solutions. Injectable preparations, for example, sterile injectable aqueous or oleaginous suspensions, may be formulated according to the known in the art by using suitable dispersing or wetting agents and suspending agents. The sterile injectable preparation may also be a sterile injectable solution or suspension in a nontoxic parenterally acceptable diluent or solvent, for example, a solution in 1,3-butanediol.

Among the acceptable vehicles and solvents that may be used are water, Ringer's solution and isotonic sodium chloride solution. In addition, sterile fixed oils are commonly used as solvents or suspending media. For this purpose, any bland fixed oil, including synthetic mono- or diglycerides, may be used. In addition, fatty acids such as oleic acid may be used in the preparation of injections.

Therefore, the compound according to the present invention may be formulated for parenteral administration (e.g., by injection, e.g., by bolus injection or continuous infusion) and presented in unit dosage form, for example, ampoules, prefilled syringes, small volume injectors or multi-dose containers, with an added preservative. The pharmaceutical compositions may take such forms as suspensions, solutions or emulsions in oily or aqueous vehicles and may contain formulatory agents, such as suspending, stabilizing and/or dispersing agents.

Alternatively, the active ingredient may be in a powder form, obtained by aseptic isolation of a sterile solid or by lyophilization from a solution, for constituting with a suitable vehicle, for example, sterile pyrogen-free water, prior to use.

Aqueous preparations that are suitable for oral use may be prepared by dissolving or suspending the active ingredient in water and adding suitable colorants, flavoring agents, stabilizers and thickeners according to the purpose.

Aqueous suspensions that are suitable for oral use may be prepared by dispersing the finely divided active ingredient in water together with a viscous substance, such as a natural or synthetic gum, resin, methylcellulose, sodium carboxymethylcellulose, or other well-known suspending agents.

Also included are solid form preparations intended to be converted to liquid form preparations for oral administration just prior to use. Such liquid forms include solutions, suspensions and emulsions. These preparations may contain, in addition to the active ingredient, colorants, flavoring agents, stabilizers, buffers, artificial and natural sweeteners, dispersants, thickeners, solubilizers and the like.

For topical administration to the epidermis, the compound according to the invention may be formulated as ointments, creams or lotions or as transdermal patches.

Ointments and creams may be formulated by using an aqueous or oily base, for example, with the addition of suitable thickening and/or gelling agents. Lotions may be formulated by using an aqueous or oily base, and will usually also contain one or more emulsifiers, stabilizers, dispersants, suspending agents, thickening agents or colorants.

Formulations that are suitable for topical administration to the mouth include lozenges including the active ingredient in a flavored base, usually sucrose and acacia or tragacanth; pastilles including the active ingredient in an inert base such as gelatin and glycerin or sucrose and acacia; and mouthwashes including the active ingredient in a suitable liquid carrier.

Solutions or suspensions are applied directly to the nasal passages by conventional means, for example, by dropper, pipette or spray.

The formulation may be provided in single or multi-dose form. In the latter case, it may be provided with a dropper, pipette or spray to enable administration to the subject in a predetermined volume of dose.

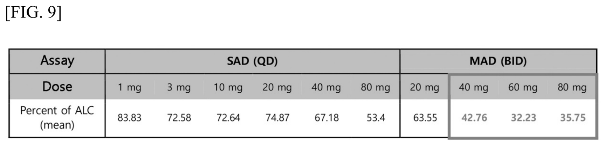

According to the present invention, in a hematological test after administration of the pharmaceutical composition, the absolute lymphocyte count decrease (decrease rate) was about 32% at 40 mg for a single administration and about 46% at 80 mg (i.e., the absolute lymphocyte count after administration compared to the absolute lymphocyte count before administration was about 68% and about 54%, respectively, and as a result, the absolute lymphocyte count can be decreased to at least about 70% of the initial level), and in the case of repeated administrations, the absolute lymphocyte count was about 57% at 40 mg, about 68% at 60 mg, and about 64% at 80 mg (i.e., the absolute lymphocyte count after administration compared to the lymphocyte count before administration was about 43%, about 32%, and about 36%, respectively, and as a result, the absolute lymphocyte count can be decreased to at least about 50% of the initial level) (FIG. 9).

Therefore, in the present invention, the absolute lymphocyte count may be suppressed to about 70% or less after administration of the pharmaceutical composition compared to before administration.

For example, compared to before administration, the pharmaceutical composition may suppress an absolute lymphocyte count after administration to about 70%, about 69%, about 68%, about 67%, about 66%, about 65%, about 64%, about 63%, about 62%, about 61%, about 60%, about 59%, about 58%, about 57%, about 56%, about 55%, about 54%, about 53%, about 52%, about 51%, about 50%, about 49%, about 48%, about 47%, about 46%, about 45%, about 44%, about 43%, about 42%, about 41%, about 40%, about 39%, about 38%, about 37%, about 36%, about 35%, about 34%, about 33%, about 32%, about 31%, or about 30% or less.

In another aspect, the pharmaceutical composition may suppress an absolute lymphocyte count to about 60% or less after administration compared to before administration.

In still another aspect, the pharmaceutical composition may suppress an absolute lymphocyte count to about 50% or less after administration compared to before administration.

In still another aspect, the pharmaceutical composition may suppress an absolute lymphocyte count to about 40% or less after administration compared to before administration.

In still another aspect, the pharmaceutical composition may suppress an absolute lymphocyte count to about 35% or less after administration compared to before administration.

In the present invention, the lymphocyte-mediated disease may be, but is not limited to, alopecia areata, inflammatory bowel disease, interstitial fibrosis and tubular atrophy or focal segmental glomerulosclerosis.

In one aspect, the lymphocyte-mediated disease is alopecia areata.

In the present invention, administration of the pharmaceutical composition may mean administration of an active ingredient in the pharmaceutical composition.

In another aspect, the present invention may be provided in the form of a kit including instructions instructing a method of administration (administration dose, administration cycle and/or administration period) to a subject in need of the prevention or treatment of a lymphocyte-mediated disease.

In still another aspect, the present invention provides an administration method including administering the active ingredient or the pharmaceutical composition to a subject in need thereof at a dose equivalent to 40 to 80 mg of a compound represented by Chemical Formula 1.

In the present invention, the administration method may be for the treatment of a lymphocyte-mediated disease, and therefore, the administration method may be a therapeutic method.

In still another aspect, the present invention relates to the use of the active ingredient or the pharmaceutical composition in the manufacture of a drug for treating a lymphocyte-mediated disease, wherein the drug is a drug for administration at a dose equivalent to 40 to 80 mg of a compound represented by Chemical Formula 1.

In the present invention, the drug may include an active ingredient at a dose equivalent to 40 to 80 mg of a compound represented by Chemical Formula 1.

In still another aspect, the present invention relates to the use of the active ingredient or the pharmaceutical composition for the treatment of a lymphocyte-mediated disease, wherein the active ingredient or the pharmaceutical composition is administered at a dose equivalent to 40 to 80 mg of the compound represented by Chemical Formula 1.

In still another aspect, the present invention relates to the active ingredient or the pharmaceutical composition for use in the treatment of a lymphocyte-mediated disease, wherein the active ingredient or the pharmaceutical composition is to be administered at a dose equivalent to 40 to 80 mg of the compound represented by Chemical Formula 1.

The detailed description of the administration method or treatment method, medicinal use and Swiss-type medicinal use is the same as the description of the pharmaceutical composition described above, unless there is a contradiction, to avoid duplicate description.

For example, the active ingredient or pharmaceutical composition may be administered according to various dosages or usage methods (frequency, cycle, period, etc.) described as suitable for administration of the active ingredient or pharmaceutical composition as described above, and the drug may be prepared so as to be administered according to various dosages or usage methods (frequency, cycle, period, etc.) described as suitable for administration of the active ingredient or pharmaceutical composition as described above.

Hereinafter, the present invention will be described in more detail through examples. These examples are only intended to illustrate the present invention, and it will be apparent to those skilled in the art that the scope of the present invention is not to be construed as being limited by these examples.

### Example 1. Phase 1 clinical trial

A randomized, double-blind, placebo-controlled, step-escalation, single- and multiple-dose phase 1 clinical trial was conducted to evaluate the safety, tolerability, and pharmacokinetic/pharmacodynamic characteristics of a compound of Chemical Formula 1 (NXC736) after oral administration to healthy adult males.

### 1-1. Drugs for clinical trials

### Clinical trial drug (NXC736 tablets) and dosage thereof

The clinical trial drug product is a white, round, film-coated tablet (1 mg, 3 mg tablet) or a yellow, round, film-coated tablet (10, 20 mg, 40 mg, 60 mg, 80 mg tablet) containing the active ingredient (main ingredient) NXC736 and excipients such as diluents, binders, disintegrants, fluidizing agents and glidants. The NXC736 clinical trial drug product was manufactured as an 84 mg film-coated tablet containing 1 mg or 3 mg of the main ingredient, or a 334 mg film-coated tablet containing 10 mg, 20 mg, 40 mg, 60 mg or 80 mg of the main ingredient, and it can be stored at room temperature (1 to 30°C) in an airtight container.

### Placebo and dosage thereof

The placebo was manufactured as a white, round, film-coated tablet weighing 84 mg or a yellow, round, film-coated tablet weighing 334 mg, containing the same amounts of diluent, binder, disintegrant, fluidizing agent and glidant as the test drug, but excluding the active ingredient NXC736, and similar to the test drug, it can be stored at room temperature (1 to 30°C) in an airtight container.

### 1-2. Test subjects

In this clinical trial, a total of 42 subjects were randomly assigned to a single-administration, and a total of 39 subjects completed the study according to the clinical trial protocol. A total of 33 subjects were assigned to a repeated-administration trial, and a total of 32 subjects completed the study according to the clinical trial protocol. The analysis groups are as shown in Table 1 and Table 2.

**[Table 1]**

| Single administration trial subjects | | | | | | | | |
|---|---|---|---|---|---|---|---|---|
| | **Placebo (N=10) n (%)** | **1 mg (N=5) n (%)** | **3 mg (N=3) n(%)** | **10 mg (N=6) n(%)** | **20 mg (N-6) n(%)** | **40 mg (N=6) n(%)** | **80 mg (N=6) n (%)** | **Total (N=42) n (%)** |
| All Randomized Set | 10 (100.00) | 5 (100.00) | 3 (100.00) | 6 (100.00) | 6 (100.00) | 6 (100.00) | 6 (100.00) | 42 (100.00) |
| Safety set (SS) | 10 (100.00) | 3 (60.00) | 3 (100.00) | 5 (83.33) | 6 (100.00) | 6 (100.00) | 6 (100.00) | 39 (92.86) |
| Pharmacokinetic (PK) Set | 10 (100.00) | 3 (60.00) | 3 (100.00) | 5(83.33) | 6 (100.00) | 6 (100.00) | 6 (100.00) | 39 (92.86) |
| Pharmacodynamic (PD) Set | 10 (100.00) | 3 (60.00) | 3 (100.00) | 5 (83.33) | 6 (100.00) | 6 (100.00) | 6 (100.00) | 39 (92.86) |

| | | | | | | | | |
|---|---|---|---|---|---|---|---|---|
| Percentages are calculated by dividing by the number of subjects in each dose group. Data source: Listing 16.2.3.1 Demographic Characteristics - All Randomized Set Report generated by program: t1021_trt_R.sas Program run date/time: 16JAN2023 09:45 | | | | | | | | |

**[Table 2]**

| Repeated administration trial subjects | | | | | | |
|---|---|---|---|---|---|---|
| | **Placebo (N=8) n (%)** | **20 mg, BID (N=6) n (%)** | **40 mg, BID (N=7) n (%)** | **60 mg, BID (N=6) n (%)** | **80 mg, BID mg, (N=6) n (%)** | **Total (N=33) n (%)** |
| All Randomized Set | 8 (100.00) | 6 (100.00) | 7 (100.00) | 6 (100.00) | 6 (100.00) | 33 (100.00) |
| Safety set (SS) | 8 (100.00) | 6 (100.00) | 6 (85.71) | 6 (100.00) | 6 (100.00) | 32 (96.97) |
| Pharmacokinetic (PK) Set | 8 (100.00) | 6 (100.00) | 6 (85.71) | 6 (100.00) | 6 (100.00) | 32 (96.97) |
| Pharmacodynamic (PD) Set | 8 (100.00) | 6 (100.00) | 6 (85.71) | 6 (100.00) | 6 (100.00) | 32 (96.97) |

| | | | | | | |
|---|---|---|---|---|---|---|
| Percentages are calculated by dividing by the number of subjects in each dose group. Data source: Listing 16.2.3.1 Demographic Characteristics - All Randomized Set Report generated by program: t1022_trt_R.sas Program run date/time: 16JAN2023 15:35 | | | | | | |

### 1-3. Administration method

### Single administration studies: 1 mg, 3 mg, 10 mg, 20 mg, 40 mg, 80 mg

The subjects were administered a single oral dose of the clinical trial drug on an empty stomach at around 9:00 AM on the day of administration after fasting for at least 10 hours. In the single-administration trial, the subjects were administered the drug and hospitalized for 8 days. After discharge, they were observed through outpatient visits until day 21. Blood was collected once before administration and at regular intervals after administration, and hematological tests were performed.

### Repeated administration studies: 20 mg twice daily, 40 mg twice daily, 60 mg twice daily, 80 mg twice daily

The subjects fasted for at least 10 hours before the first administration. Afterwards, the clinical trial drug was administered orally twice a day for 13 days and once on day 14 in a fasting state at around 9:00 AM and 9:00 PM. The administration was performed twice a day for approximately 14 days, and the subjects were discharged on day 21 from the first administration and observed through outpatient visits until day 36. Blood was collected once before administration and at regular intervals after administration, and hematological tests were performed.

### 1-4. Safety test results

No serious adverse reactions were observed in single-administration and repeated-administration trials. Most adverse reactions were mild and all recovered during the trial period.

In the single-administration trial, 23 adverse reactions occurred in 9 out of 39 subjects who received the investigational drug, and 12 of these adverse reactions were adverse reactions (ADR) that the investigators assessed as having a causal relationship with the investigational drug. There were no adverse reactions that required discontinuation of the dose increase, and no serious adverse reactions occurred. Fisher's exact test confirmed that there was no significant difference in the frequency of adverse reactions between the dose groups.

As expected from the drug's mechanism of action, the decrease in heart rate after administration was observed in most subjects administered NXC736, and the degree increased with increasing administration dose. However, this phenomenon was confirmed to be alleviated with repeated administrations compared to single administration. In addition, most of the observed bradycardia had no clinical significance, and all cases collected as adverse reactions were asymptomatic. As a result of comprehensively evaluating the results of physical examination, vital signs, 12-lead electrocardiogram, pulmonary function test and ophthalmological examination, it was confirmed that the safety and tolerability were excellent up to 14 days of administration at a dose of 80 mg twice daily.

Adverse reactions that occurred in each dose group were organized according to the system organ (SOC) and preferred term (PT).

**[Table 3]**

| Treatment-emergent adverse events (TEAEs) by SOC and PT - Single ascending dose trial (SAD) | | | | | | | | |
|---|---|---|---|---|---|---|---|---|
| **System Organ Class Preferred Term** | | **Placebo (N=10)** | **1 mg (N=3)** | **3 mg (N=3)** | **10 mg (N=5)** | **20 mg (N=6)** | **40 mg (N=6)** | **80 mg (N=6)** |
| | | **n (%) [Evests]** | **n (%) [Events]** | **n (%) [Events]** | **n (%) [Events]** | **n (%) [Events]** | **n (%) [Events]** | **n (%) [Events]** |
| **Subjects with TEAEs** | | **4 (40.00) [8]** | **1 (33.33) [2]** | **0 (0.00) [0]** | **1 (20.00) [4]** | **1 (16.67) [3]** | **1(16.67) [2]** | **1 (16.67) [4]** |
| **Investigations** | | **2 (20.00) [3]** | **1 (33.33) [1]** | **0 (0.00) [0]** | **1 (20.00) [3]** | **0 (0.00) [0]** | **0 (0.00) [0]** | **0 (0.00) [0]** |
| | Blood bilirubin increased | 0 (0.00) [0] | 1 (33.33) [1] | 0 (0.00) [0] | 1 (20.00) [1] | 0 (0.00) [0] | 0 (0.00) [0] | 0 (0.00) [0] |
| | Blood creatine phosphokinase increased | 1 (10.00) [1] | 0 (0.00) [0] | 0 (0.00) [0] | 1 (20.00) [1] | 0 (0.00) [0] | 0 (0.00) [0] | 0 (0.00) [0] |
| | Liver function test increased | 1 (10.00) [1] | 0 (0.00) [0] | 0 (0.00) [0] | 1 (20.00) [1] | 0 (0.00) [0] | 0 (0.00) [0] | 0 (0.00) [0] |
| | Weight decreased | 1 (10.00) [1] | 0 (0.00) [0] | 0 (0.00) [0] | 0 (0.00) [0] | 0 (0.00) [0] | 0 (0.00) [0] | 0 (0.00) [0] |

| **General disorders and administration site conditions** | | **1 (10.00) [1]** | **0 (0.00) [0]** | **0 (0.00) [0]** | **0 (0.00) [0]** | **1 (16.67) [1]** | **1 (16.67) [1]** | **0 (0.00) [0]** |
|---|---|---|---|---|---|---|---|---|
| | Chest discomfort | 1 (10.00) [1] | 0 (0.00) [0] | 0 (0.00) [0] | 0 (0.00) [0] | 1 (16.67) [1] | 0 (0.00) [0] | 0 (0.00) [0] |
| | Pyrexia | 0 (0.00) [0] | 0 (0.00) [0] | 0 (0.00) [0] | 0 (0.00) [0] | 0 (0.00) [0] | 1 (16.67) [1] | 0 (0.00) [0] |

| **Injury, poisoning and procedural complications** | | **2 (20.00) [2]** | **0 (0.00) [0]** | **0 (0.00) [0]** | **0 (0.00) [0]** | **0 (0.00) [0]** | **0 (0.00) [0]** | **1 (16.67) [1]** |
|---|---|---|---|---|---|---|---|---|
| | Hand fracture | 1 (10.00) [1] | 0 (0.00) [0] | 0 (0.00) [0] | 0 (0.00) [0] | 0 (0.00) [0] | 0 (0.00) [0] | 0 (0.00) [0] |
| | Skin abrasion | 0 (0.00) [0] | 0 (0.00) [0] | 0 (0.00) [0] | 0 (0.00) [0] | 0 (0.00) [0] | 0 (0.00) [0] | 1 (16.67) [1] |
| | Skin laceration | 1 (10.00) [1] | 0 (0.00) [0] | 0 (0.00) [0] | 0 (0.00) [0] | 0 (0.00) [0] | 0 (0.00) [0] | 0 (0.00) [0] |

| **Cardiac disorders** | | **0 (0.00) [0]** | **0 (0.00) [0]** | **0 (0.00) [0]** | **0 (0.00) [0]** | **0 (0.00) [0]** | **1 (16.67) [1]** | **1 (16.67) [2]** |
|---|---|---|---|---|---|---|---|---|
| | Atrioventricular block first degree | 0 (0.00) [0] | 0 (0.00) [0] | 0 (0.00) [0] | 0 (0.00) [0] | 0 (0.00) [0] | 0 (0.00) [0] | 1 (16.67) [1] |
| | Atrioventricular block second degree | 0 (0.00) [0] | 0 (0.00) [0] | 0 (0.00) [0] | 0 (0.00) [0] | 0 (0.00) [0] | 0 (0.00) [0] | 1 (16.67) [1] |
| | Bradycardia | 0 (0.00) [0] | 0 (0.00) [0] | 0 (0.00) [0] | 0 (0.00) [0] | 0 (0.00) [0] | 1 (16.67) [1] | 0 (0.00) [0] |

| **Respiratory, thoracic and mediastinal disorders** | | **1 (10.00) [2]** | **0 (0.00) [0]** | **0 (0.00) [0]** | **0 (0.00) [0]** | **1 (16.67) [1]** | **0 (0.00) [0]** | **0 (0.00) [0]** |
|---|---|---|---|---|---|---|---|---|
| | Epistaxis | 1 (10.00) [1] | 0 (0.00) [0] | 0 (0.00) [0] | 0 (0.00) [0] | 0 (0.00) [0] | 0 (0.00) [0] | 0 (0.00) [0] |
| | Nasal congestion | 1 (10.00) [1] | 0 (0.00) [0] | 0 (0.00) [0] | 0 (0.00) **[0]** | 0 (0.00) [0] | 0 (0.00) [0] | 0 (0.00) [0] |

| **System Organ Class" Preferred Term** | | **Placebo (N=10)** | **1 mg (N=3)** | **3 mg (N=3)** | **10 mg (N=5)** | **20 mg (N=6)** | **40 mg (N=6)** | **80 mg (N=6):** |
|---|---|---|---|---|---|---|---|---|
| | | **n (%) [Events]** | **n (%) [Events]** | **[Events]** | **n(%) [Events]** | **n(%) [Events]** | **n (%) [Events]** | **n (%) [Events]** |
| | Productive cough | 0 (0.00) [0] | 0 (0.00) [0] | 0 (0.00) [0] | 0 (0.00) [0] | 1 (16.67) [1] | 0 (0.00) [0] | 0 (0.00) [0] |

| **Gastrointestinal disorders** | | **0 (0.00) [0]** | **0 (0.00) [0]** | **0 (0.00) [0]** | **1 (20.00) [1]** | **1 (16.67) [1]** | **0 (0.00) [0]** | **0 (0.00) [0]** |
|---|---|---|---|---|---|---|---|---|
| | Cheilitis | 0 (0.00) [0] | 0 (0.00) [0] | 0 (0.00) [0] | 1 (20.00) [1] | 0 (0.00) [0] | 0 (0.00) [0] | 0 (0.00) [0] |
| | Lip blister | 0 (0.00) [0] | 0 (0.00) [0] | 0 (0.00) [0] | 0 (0.00) [0] | 1 (16.67) [1] | 0 (0.00) [0] | 0 (0.00) [0] |

| **Eye disorders** | | **0 (0.00) [0]** | **1 (33.33) [1]** | **0 (0.00) [0]** | **0 (0.00) [0]** | **0 (0.00) [0]** | **0 (0.00) [0]** | **0 (0.00) [0]** |
|---|---|---|---|---|---|---|---|---|
| | Swelling of eyelid | 0 (0.00) [0] | 1 (33.33) [1] | 0 (0.00) [0] | 0 (0.00) [0] | 0 (0.00) [0] | 0 (0.00) [0] | 0 (0.00) [0] |

| **Nervous system disorders** | | **0 (0.00) [0]** | **0 (0.00) [0]** | **0 (0.00) [0]** | **0 (0.00) [0]** | **0 (0.00) [0]** | **0 (0.00) [0]** | **1 (16.67) [1]** |
|---|---|---|---|---|---|---|---|---|
| | Dizziness | 0 (0.00) [0] | 0 (0.00) [0] | 0 (0.00) [0] | 0 (0.00) [0] | 0 (0.00) [0] | 0 (0.00) [0] | 1 (16.67) [1] |

| | | | | | | | | |
|---|---|---|---|---|---|---|---|---|
| Percentages are calculated by dividing by the number of subjects in each cohort. Subjects may have more than one adverse event per SOC and PT. MedDRA version 25.0 Data source: Listing 16.2.5 Treatment-Emergent Adverse Event (TEAE) - Safety Set Report generated by program: t30221_ac S.sas Program run date/time: 16JAN2023 11:04 | | | | | | | | |

**[Table 4]**

| Adverse drug reactions (ADRs) by SOC and PT - Single ascending dose trial (SAD) | | | | | | | | |
|---|---|---|---|---|---|---|---|---|
| | **System Organ Class Preferred Term** | **Placebo (N-10)** | **1 mg (N=3)** | **3 mg (N=3)** | **10 mg (N=5)** | **20 mg (N=6) %n** | **40 mg** | **80 mg (N=6)** |
| | | **n(%) [Events]** | **n (%) [Events]** | **n (%) [Events]** | **n (%) [Events]** | **[Events]** | **n (%) [Events]** | **n (%) [Events]** |
| **Subjects with ADRs** | | **2 (20.00) [4]** | **1 (33.33) [1]** | **0 (0.00) [0]** | **1 (20.00) [1]** | **1 (16.67) [2]** | **1 (16.67) [1]** | **1 (16.67) [3]** |
| **Investigations** | | **1 (10.00) [1]** | **1 (33.33) [1]** | **0 (0.00) [0]** | **1 (20.00) [1]** | **0 (0.00) [0]** | **0 (0.00) [0]** | **0 (0.00) [0]** |
| | Blood bilirubin increased | 0 (0.00) [0] | 1 (33.33) [1] | 0 (0.00) [0] | 1 (20.00) [1] | 0 (0.00) [0] | 0 (0.00) [0] | 0 (0.00) [0] |
| | Weight decreased | 1 (10.00) [1] | 0 (0.00) [0] | 0 (0.00) [0] | 0 (0.00) [0] | 0 (0.00) [0] | 0 (0.00) [0] | 0 (0.00) [0] |

| **Cardiac disorders** | | **0 (0.00) [0]** | **0 (0.00) [0]** | **0 (0.00) [0]** | **0 (0.00) [0]** | **0 (0.00) [0]** | **1 (16.67) [1]** | **1 (16.67) [2]** |
|---|---|---|---|---|---|---|---|---|
| | Atrioventricular block first degree | 0 (0.00) [0] | 0 (0.00) [0] | 0 (0.00) [0] | 0 (0.00) [0] | 0 (0.00) [0] | 0 (0.00) [0] | 1 (16.67) [1] |
| | Atrioventricular block second degree | 0 (0.00) [0] | 0 (0.00) [0] | 0 (0.00) [0] | 0 (0.00) [0] | 0 (0.00) [0] | 0 (0.00) [0] | 1 (16.67) [1] |
| | Bradycardia | 0 (0.00) [0] | 0 (0.00) [0] | 0 (0.00) [0] | 0 (0.00) [0] | 0 (0.00) [0] | 1 (16.67) [1] | 0 (0.00) [0] |

| **Respiratory, thoracic and mediastinal disorders** | | **1 (10.00) [2]** | **0 (0.00) [0]** | **0 (0.00) [0]** | **0 (0.00) [0]** | **1 (16.67) [1]** | **0 (0.00) [0]** | **0 (0.00) [0]** |
|---|---|---|---|---|---|---|---|---|
| | Epistaxis | 1 (10.00) [1] | 0 (0.00) [0] | 0 (0.00) [0] | 0 (0.00) [0] | 0 (0.00) [0] | 0 (0.00) [0] | 0 (0.00) [0] |
| | Nasal congestion | 1 (10.00) [1] | 0 (0.00) [0] | 0 (0.00) [0] | 0 (0.00) [0] | 0 (0.00) [0] | 0 (0.00) [0] | 0 (0.00) [0] |
| | Productive cough | 0 (0.00) [0] | 0 (0.00) [0] | 0 (0.00) [0] | 0 (0.00) [0] | 1 (16.67) [1] | 0 (0.00) [0] | 0 (0.00) [0] |

| **General disorders and administration site conditions** | | **1 (10.00) [1]** | **0 (0.00) [0]** | **0 (0.00) [0]** | **0 (0.00) [0]** | **1 (16.67) [1]** | **0 (0.00) [0]** | **0 (0.00) [0]** |
|---|---|---|---|---|---|---|---|---|
| | Chest discomfort | 1 (10.00) [1] | 0 (0.00) [0] | 0 (0.00) [0] | 0 (0.00) [0] | 1 (16.67) [1] | 0 (0.00) [0] | 0 (0.00) [0] |

| **Nervous system disorders** | | **0 (0.00) [0]** | **0 (0.00) [0]** | **0 (0.00) [0]** | **0 (0.00) [0]** | **0 (0.00) [0]** | **0 (0.00) [0]** | **1 (16.67) [1]** |
|---|---|---|---|---|---|---|---|---|
| | Dizziness | 0 (0.00) [0] | 0 (0.00) [0] | 0 (0.00) [0] | 0 (0.00) [0] | 0 (0.00) [0] | 0 (0.00) [0] | 1 (16.67) [1] |

| | | | | | | | | |
|---|---|---|---|---|---|---|---|---|
| Percentages are calculated by dividing by the number of subjects in each cohort. Subjects may have more than one adverse event per SOC and PT. MedDRA version 25.0 Data source: Listing 16.2.5 Treatment-Emergent Adverse Event (TEAE) - Safety Set Report generated by program: t30231_ae_S.sas Program run date/time: 24FEB2023 08:11 | | | | | | | | |

**[Table 5]**

| Treatment-emergent adverse events (TEAEs) by SOC and PT - Multiple ascending dose trial (MAD) | | | | | | |
|---|---|---|---|---|---|---|
| **System Organ Class** | | **Placebo (N=8)** | **20 mg, BID (N=6)** | **40 mg, BID (N=6)** | **60 mg, BID (N=6)** | **80 mg, BED (N=6)** |
| | **Preferred Term** | | | | | |
| | | **n (%) [Events]** | **n (%) [Events]** | **n (%) [Events]** | **n (%) [Events]** | **n (%) [Events]** |
| **Subjects with TEAEs** | | **3 (37.50) [3]** | **3 (50.00) [5]** | **1 (16.67) [4]** | **4 (66.67) [8]** | **5 (83.33) [8]** |
| **Investigations** | | **1 (12.50) [1]** | **1 (16.67) [3]** | **0 (0.00) [0]** | **3 (50.00) [3]** | **2 (33.33) [2]** |
| | White blood cell count decreased | 0 (0.00) [0] | 0 (0.00) [0] | 0 (0.00) [0] | 2 (33.33) [2] | 0 (0.00) [0] |
| | Aspartate aminotransferase increased | 0 (0.00) [0] | 1 (16.67) [1] | 0 (0.00) [0] | 0 (0.00) [0] | 0 (0.00) [0] |
| | Blood bilirubin increased | 0 (0.00) [0] | 0 (0.00) [0] | 0 (0.00) [0] | 0 (0.00) [0] | 1 (16.67) [1] |
| | Blood creatine phosphokinase increased | 0 (0.00) [0] | 1 (16.67) [1] | 0 (0.00) [0] | 0 (0.00) [0] | 0 (0.00) [0] |
| | Blood lactate dehydrogenase increased | 0 (0.00) [0] | 1 (16.67) [1] | 0 (0.00) [0] | 0 (0.00) [0] | 0 (0.00) [0] |
| | Blood phosphorus increased | 1 (12.50) [1] | 0 (0.00) [0] | 0 (0.00) [0] | 0 (0.00) [0] | 0 (0.00) [0] |
| | Blood triglycerides increased | 0 (0.00) [0] | 0 (0.00) [0] | 0 (0.00) [0] | 0 (0.00) [0] | 1 (16.67) [1] |
| | Gamma-glutamyltransferase increased | 0 (0.00) [0] | 0 (0.00) [0] | 0 (0.00) [0] | 1 (16.67) [1] | 0 (0.00) [0] |
| **Cardiac disorders** | | **0 (0.00) [0]** | **0 (0.00) [0]** | **0 (0.00) [0]** | **2 (33.33) [2]** | **1 (16.67) [1]** |
| | Bradycardia | 0 (0.00) [0] | 0 (0.00) [0] | 0 (0.00) [0] | 2 (33.33) [2] | 1 (16.67) [1] |
| **General disorders and administration site conditions** | | **1 (12.50) [1]** | **1 (16.67) [1]** | **0 (0.00) [0]** | **0 (0.00) [0]** | **1 (16.67) [1]** |
| | Chest pain | 1 (12.50) [1] | 0 (0.00) [0] | 0 (0.00) [0] | 0 (0.00) [0] | 0 (0.00) [0] |
| | Influenza like illness | 0 (0.00) [0] | 0 (0.00) [0] | 0 (0.00) [0] | 0 (0.00) [0] | 1 (16.67) [1] |
| | Sensation of foreign body | 0 (0.00) [0] | 1 (16.67) [1] | 0 (0.00) [0] | 0 (0.00) [0] | 0 (0.00) [0] |
| **Gastrointestinal disorders** | | **0 (0.00) [0]** | **0 (0.00) [0]** | **1 (16.67) [2]** | **0 (0.00) [0]** | **1 (16.67) [1]** |
| | Diarrhoea | 0 (0.00) [0] | 0 (0.00) [0] | 1 (16.67) [1] | 0 (0.00) [0] | 0 (0.00) [0] |
| | Nausea | 0 (0.00) [0] | 0 (0.00) [0] | 1 (16.67) [1] | 0 (0.00) [0] | 0 (0.00) [0] |
| | Parotid gland enlargement | 0 (0.00) [0] | 0 (0.00) [0] | 0 (0.00) [0] | 0 (0.00) [0] | 1 (16.67) [1] |
| **Respiratory, thoracic and mediastinal disorders** | | **0 (0.00) [0]** | **0 (0.00) [0]** | **1 (16.67) [1]** | **0 (0.00) [0]** | **1 (16.67) [1]** |
| | Dyspnoea | 0 (0.00) [0] | 0 (0.00) [0] | 1 (16.67) [1] | 0 (0.00) [0] | 0 (0.00) [0] |
| | Oropharyngeal pain | 0 (0.00) [0] | 0 (0.00) [0] | 0 (0.00) [0] | 0 (0.00) [0] | 1 (16.67) [1] |
| **Skin and subcutaneous tissue disorders** | | **1 (12.50) [1]** | **0 (0.00) [0]** | **1 (16.67) [1]** | **0 (0.00) [0]** | **0 (0.00) [0]** |
| | Cold sweat | 0 (0.00) [0] | 0 (0.00) [0] | 1 (16.67) [1] | 0 (0.00) [0] | 0 (0.00) [0] |
| | Dermatitis contact | 1 (12.50) [1] | 0 (0.00) [0] | 0 (0.00) [0] | 0 (0.00) [0] | 0 (0.00) [0] |
| **Eye disorders** | | **0 (0.00) [0]** | **0 (0.00) [0]** | **0 (0.00) [0]** | **1 (16.67) [3]** | **0 (0.00) [0]** |
| | Eye pruritus | 0 (0.00) [0] | 0 (0.00) [0] | 0 (0.00) [0] | 1 (16.67) [1] | 0 (0.00) [0] |
| | Foreign body sensation in eyes | 0 (0.00) [0] | 0 (0.00) [0] | 0 (0.00) [0] | 1 (16.67) [1] | 0 (0.00) [0] |
| | Ocular hyperaemia | 0 (0.00) [0] | 0 (0.00) [0] | 0 (0.00) [0] | 1 (16.67) [1] | 0 (0.00) [0] |
| **Infections and infestations** | | **0 (0.00) [0]** | **0 (0.00) [0]** | **0 (0.00) [0]** | **0 (0.00) [0]** | **1 (16.67) [1]** |
| | Upper respiratory tract infection | 0 (0.00) [0] | 0 (0.00) [0] | 0 (0.00) [0] | 0 (0.00) [0] | 1 (16.67) [1] |

| **System Organ Class** | | **Placebo (N=8)** | **20 mg, BID (**N**=6)** | **40 mg, BID (N=6)** | **60 mg, BID** | **80 mg, BID (N=6)** |
|---|---|---|---|---|---|---|
| | **Preferred Term** | | | | | |
| | | **n (%) [Events]** | **n (%) [Events]** | **n (%) [Events]** | **(N=6) n (%) [Events]** | **n (%) [Events]** |
| **Injury, poisoning and procedural complications** | | **0 (0.00) [0]** | **1 (16.67) [1]** | **0 (0.00) [0]** | **0 (0.00) [0]** | **0 (0.00) [0]** |
| | Lip injury | 0 (0.00) [0] | 1 (16.67) [1] | 0 (0.00) [0] | 0 (0.00) [0] | 0 (0.00) [0] |
| **Nervous system disorders** | | **0 (0.00) [0]** | **0 (0.00) [0]** | **0 (0.00) [0]** | **0 (0.00) [0]** | **1 (16.67) [1]** |
| | Headache | 0 (0.00) [0] | 0 (0.00) [0] | 0 (0.00) [0] | 0 (0.00) [0] | 1 (16.67) [1] |

| | | | | | | |
|---|---|---|---|---|---|---|
| Percentages are calculated by dividing by the number of subjects in each cohort. Subjects may have more than one adverse event per SOC and PT. MedDRA version 25.0 Data source: Listing 16.2.5 Treatment-Emergent Adverse Event (TEAE) - Safety Set Report generated by program: t30222_ae_S.sas Program run date/time: 16JAN2023 15:43 | | | | | | |

**[Table 6]**

| Adverse drug reactions (ADRs) by SOC and PT - Multiple ascending dose trial (MAD) | | | | | | |
|---|---|---|---|---|---|---|
| **System Organ Class** | | **Placebo (N-8)** | **20 mg, BID (N=6)** | **40 mg, BID (N=6)** | **60 mg, BID (N=6)** | **80 mg, BID (N=6)** |
| | **Preferred Term** | | | | | |
| | | **n (%) [Events]** | **n (%) [Events]** | **n (%) [Events]** | **n (%) [Events]** | **n (%) [Events]** |
| **Subjects with ADRs** | | **1 (12.50) [1]** | **1 (16.67) [3]** | **0 (0.00) [0]** | **4 (66.67) [4]** | **3 (50.00) [6]** |
| **Investigations** | | **0 (0.00) [0]** | **1 (16.67) [3]** | **0 (0.00) [0]** | **2 (33.33) [2]** | **1 (16.67) [1]** |
| | White blood cell count decreased | 0 (0.00) [0] | 0 (0.00) [0] | 0 (0.00) [0] | 2 (33.33) [2] | 0 (0.00) [0] |
| | Aspartate aminotransferase increased | 0 (0.00) [0] | 1 (16.67) [1] | 0 (0.00) [0] | 0 (0.00) [0] | 0 (0.00) [0] |
| | Blood bilirubin increased | 0 (0.00) [0] | 0 (0.00) [0] | 0 (0.00) [0] | 0 (0.00) [0] | 1 (16.67) [1] |
| | Blood creatine phosphokinase increased | 0 (0.00) [0] | 1 (16.67) [1] | 0 (0.00) [0] | 0 (0.00) [0] | 0 (0.00) [0] |
| | Blood lactate dehydrogenase increased | 0 (0.00) [0] | 1 (16.67) [1] | 0 (0.00) [0] | 0 (0.00) [0] | 0 (0.00) [0] |
| **Cardiac disorders** | | **0 (0.00) [0]** | **0 (0.00) [0]** | **0 (0.00) [0]** | **2 (33.33) [2]** | **1 (16.67) [1]** |
| | Bradycardia | 0 (0.00) [0] | 0 (0.00) [0] | 0 (0.00) [0] | 2 (33.33) [2] | 1 (16.67) [1] |
| **Gastrointestinal disorders** | | **0 (0.00) [0]** | **0 (0.00) [0]** | **0 (0.00) [0]** | **0 (0.00) [0]** | **1 (16.67) [1]** |
| | Parotid gland enlargement | 0 (0.00) [0] | 0 (0.00) [0] | 0 (0.00) [0] | 0 (0.00) [0] | 1 (16.67) [1] |
| **General disorders and administration site conditions** | | **1 (12.50) [1]** | **0 (0.00) [0]** | **0 (0.00) [0]** | **0 (0.00) [0]** | **0 (0.00) [0]** |
| | Chest pain | 1 (12.50) [1] | 0 (0.00) [0] | 0 (0.00) [0] | 0 (0.00) [0] | 0 (0.00) [0] |
| **Infections and infestations** | | **0 (0.00) [0]** | **0 (0.00) [0]** | **0 (0.00) [0]** | **0 (0.00) [0]** | **1 (16.67) [1]** |
| | Upper respiratory tract infection | 0 (0.00) [0] | 0 (0.00) [0] | 0 (0.00) [0] | 0 (0.00) [0] | 1 (16.67) [1] |
| **Nervous system disorders** | | **0 (0.00) [0]** | **0 (0.00) [0]** | **0 (0.00) [0]** | **0 (0.00) [0]** | **1 (16.67) [1]** |
| | Headache | 0 (0.00) [0] | 0 (0.00) [0] | 0 (0.00) [0] | 0 (0.00) [0] | 1 (16.67) [1] |
| **Respiratory, thoracic and mediastinal disorders** | | **0 (0.00) [0]** | **0 (0.00) [0]** | **0 (0.00) [0]** | **0 (0.00) [0]** | **1 (16.67) [1]** |
| | Oropharyngeal pain | 0 (0.00) [0] | 0 (0.00) [0] | 0 (0.00) [0] | 0 (0.00) [0] | 1 (16.67) [1] |

| | | | | | | |
|---|---|---|---|---|---|---|
| Percentages are calculated by dividing by the number of subjects in each cohort. Subjects may have more than one adverse event per SOC and PT. MedDRA version 25.0 Data source: Listing 16.2.5 Treatment-Emergent Adverse Event (TEAE) - Safety Set Report generated by program: t30232_ae_S.sas Program run date/time: 24FEB2023 08:12 | | | | | | |

### 1-5. Pharmacokinetic test results

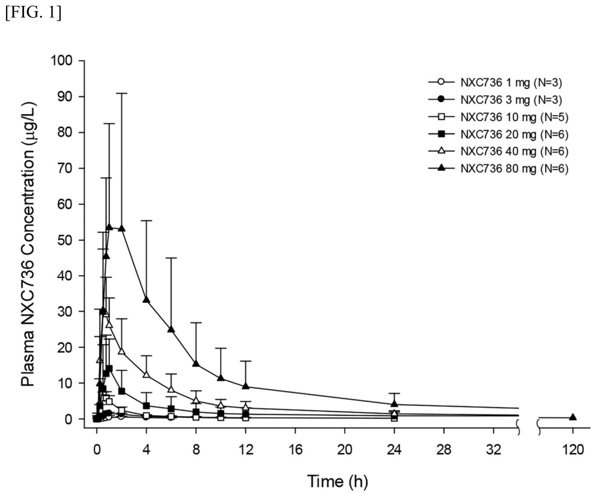

The clinical trial drug was rapidly absorbed into the body after a single administration, reaching peak plasma concentrations in about 1 hour. The average elimination half-life was about 10 hours, and it was hardly eliminated in urine (FIG. 1).

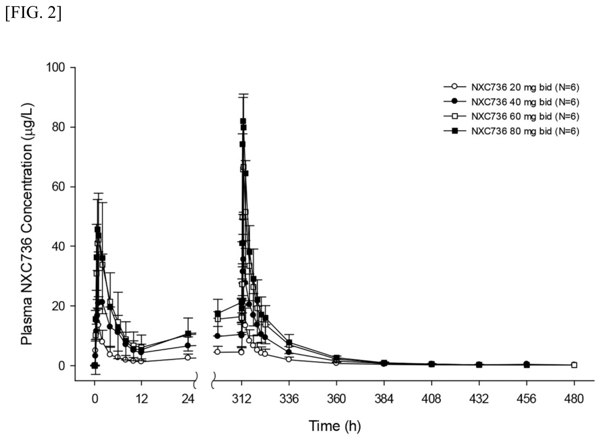

Meanwhile, in the repeated administration trial, the clinical trial drug accumulated approximately twice as much at steady state compared to the first administration (FIG. 2).

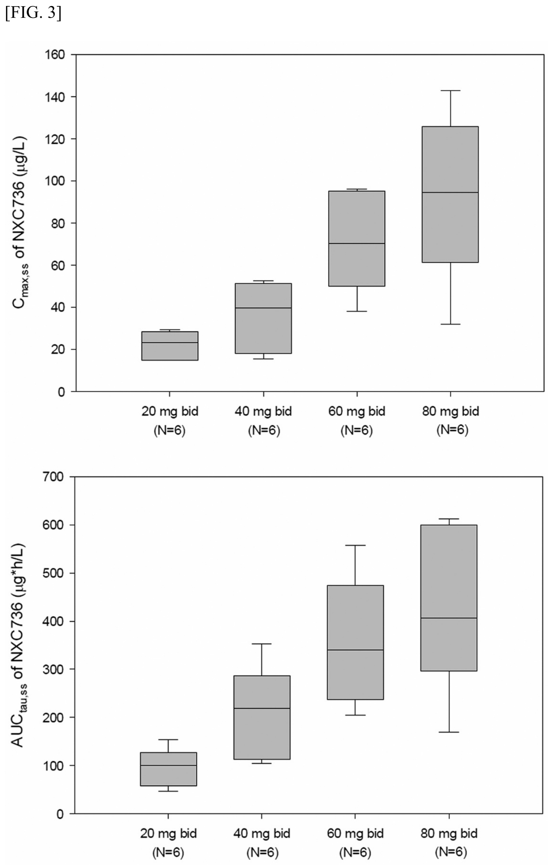

After repeated administrations of the clinical trial drug, the dose proportionality of Cmax,ss and AUCtau,ss was confirmed (FIG. 3).

### 1-6. Pharmacodynamic test results

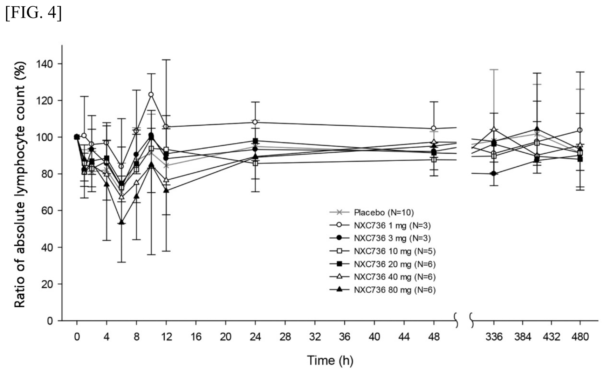

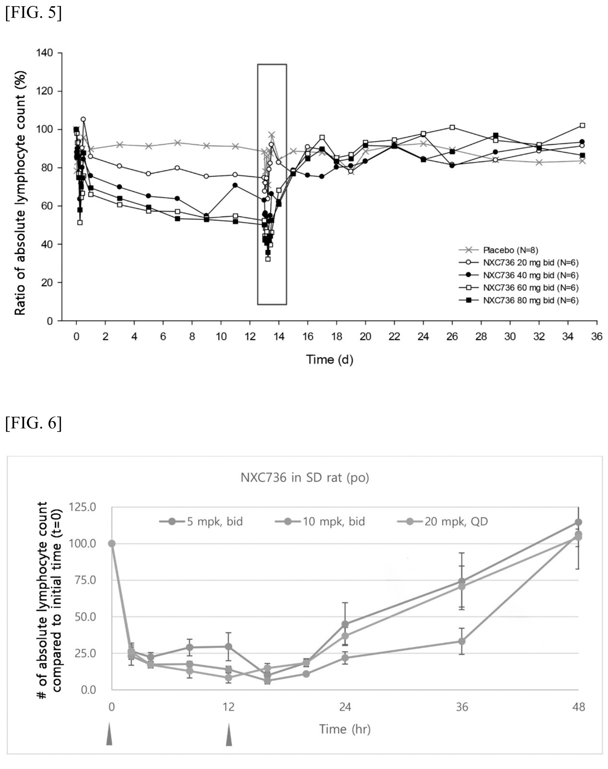

In both single-administration and repeated-administration trials, the absolute lymphocyte count (ALC) steadily decreased for up to 6 hours after administration, but it quickly recovered to the pre-administration level within about 1 day after single administration and within about 1 to 2 days after repeated administrations. In the single-administration trial, the absolute lymphocyte count decreased by only about 46% at the highest dose of 80 mg, but in the repeated-administration trial, the absolute lymphocyte count decreased by up to 70% in the 60 mg twice-daily administration group or higher after repeated administrations for 14 days. In the single-administration trial, the correlation between the drug and systemic exposure-pharmacodynamic parameters was relatively unclear due to the relatively long observation period compared to the actual action time of the drug, but in the repeated-administration trial, an increase in the pharmacodynamic parameters was clearly observed when the drug was administered twice a day at doses of 20 to 60 mg, and a strong correlation was also observed with systemic exposure. When administered at a dose of 80 mg twice daily, the maximum absolute lymphocyte count reduction was approximately 60 to 70%, similar to that when administered at a dose of 60 mg twice daily. This confirmed that the pharmacological action was saturated at doses higher than 60 mg twice daily (FIG. 4, FIG. 5).

### Example 2. Determination of pharmaceutically effective dose

Alopecia areata is an autoimmune disease induced by T lymphocytes and it is known that hair loss occurs when T lymphocytes attack hair follicles due to the destruction of the immune privilege protecting the hair follicles. Therefore, the suppression of these T lymphocytes is known as a method of treating alopecia areata, and JAK inhibitors, Baricitinib (Olumiant) and Ritlecitinib (Litfulo), show a therapeutic effect on alopecia areata through the suppression of T lymphocytes (doi.org/10.3389/fimmu.2023.1243556).

### 2-1. Confirmation of the absolute lymphocyte count suppression effect following the administration of NXC736 in healthy rats

In order to determine the effect of NXC736 on the inhibition of lymphocyte changes in rats (SD rats, Koatech) after single oral administration of 20 mg/kg once daily (QD) or oral administration of 5 mg/kg or 10 mg/kg twice daily (1 additional administration 12 hours after the first administration) (BID), lymphocytes in the peripheral blood stream were measured for a total of 10 times at 0, 2, 4, 8, 12, 16, 20, 24, 36 and 48 hours, with the first administration time as time 0 by using an automated hematocytometer (Advia 2020i, SIEMENS).

When administered orally once a day at a dose of 20 mg/kg, lymphocytes were suppressed by up to 92% in 12 hours, but recovered to 60% in 24 hours, and 100% in 48 hours. Meanwhile, when administered orally twice a day at a dose of 5 mg/kg, lymphocytes were suppressed by up to 78% in 4 hours after the first administration, suppressed by approximately 90% in 4 hours after the second administration (16 hours after the first administration), recovered to approximately 50% in 24 hours, and then recovered to the pre-administration state. In addition, when administered orally twice a day at a dose of 10 mg/kg, it was confirmed that lymphocytes were suppressed by up to 83% 4 hours after the first administration, and up to 94% 4 hours after the second administration (i.e., 16 hours after the first administration), and that the suppression effect of about 80% was maintained for up to 24 hours and about 70% was maintained for up to 36 hours (FIG. 6).

From the above results, it was confirmed that the lymphocyte suppression effect was superior when administered twice a day even at a lower dose compared to when administered once a day.

### 2-2. Confirmation of the inhibitory effect on alopecia areata using the C3H/HeJ alopecia areata mouse model

The efficacy of NXC736 on alopecia areata was evaluated by using the alopecia areata mouse model C3H/HeJ (Jackson Lab, Saeron Bio). C3H/HeJ mice are experimental animals that naturally develop alopecia areata as they age, and lymph node cells obtained from C3H/HeJ mice over 20 weeks old in which alopecia areata is naturally induced can be proliferated and injected into C3H/HeJ mice 10 to 16 weeks old in which alopecia areata is not induced, thereby inducing alopecia areata.

C3H/HeJ mice, 10 to 16 weeks of age, in which alopecia areata was not induced, were divided into two groups: a control group (sterile distilled water administration group) and an NXC736 administration experimental group, and 5 mice per group were randomly assigned. After the lymph node cells were proliferated and injected into the C3H/HeJ mice, the NXC736 administration experimental group was orally administered with 3 mg/kg or 10 mg/kg of NXC736, and the control group was orally administered with the same volume of sterile distilled water twice a day at the same time for 12 weeks. The alopecia areata lesion area and disease free ratio were measured at two-week intervals. The animal breeding room was maintained at a temperature of 23±3°C, a relative humidity of 50±10%, and a 12-hour light and dark cycle, and the experimental animals were bred in isolation boxes (ThreeShine) and were given experimental animal feed and water ad libitum.

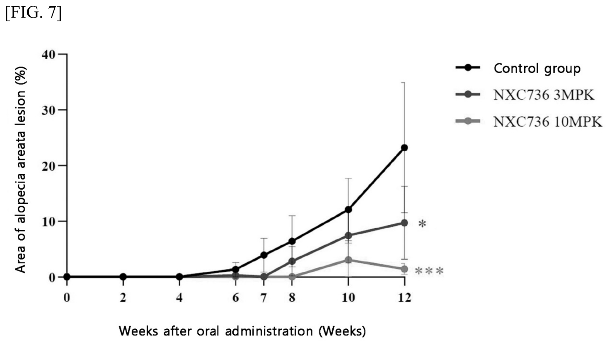

After 12 weeks, the circular alopecia lesion area was photographed and quantified by using the Image J program (Version 1.44p). As a result, the circular alopecia lesion area was effectively reduced in the NXC736-administered experimental group compared to the circular alopecia lesion area in the control group, and particularly, a concentration-dependent therapeutic effect was observed according to NXC736 administration (FIG. 7).

### 2-3. Determination of pharmaceutical effective dose through ALC measurement

As a result of measuring ALC through hematology tests in phase 1 clinical trials, it was confirmed that ALC decreased to the maximum at about 6 hours after single administration and recovered to the pre-administration level within the same day. In the single-administration trial, the change in ALC was 16% at 1 mg, 27% at 3 mg, 27% at 10 mg, 25% at 20 mg and 32% at 40 mg, and even at the highest dose of 80 mg, it decreased only to a maximum of 46%.

However, in repeated administrations, it was confirmed that the maximum decrease in ALC during the administration interval gradually decreased as the administration was repeated and reached the maximum within 14 days, and this was also confirmed to recover to the pre-administration level within 24 hours after discontinuation of administration. In the 14-day repeated administration trial, the change in ALC decreased only to a maximum of 30% in the 20 mg twice daily administration group, similar to the single administration, but decreased by up to 57% or more in the 40 mg twice daily administration group, and by about 60% or more and up to close to 70% in the 60 mg and 80 mg twice daily administration groups.

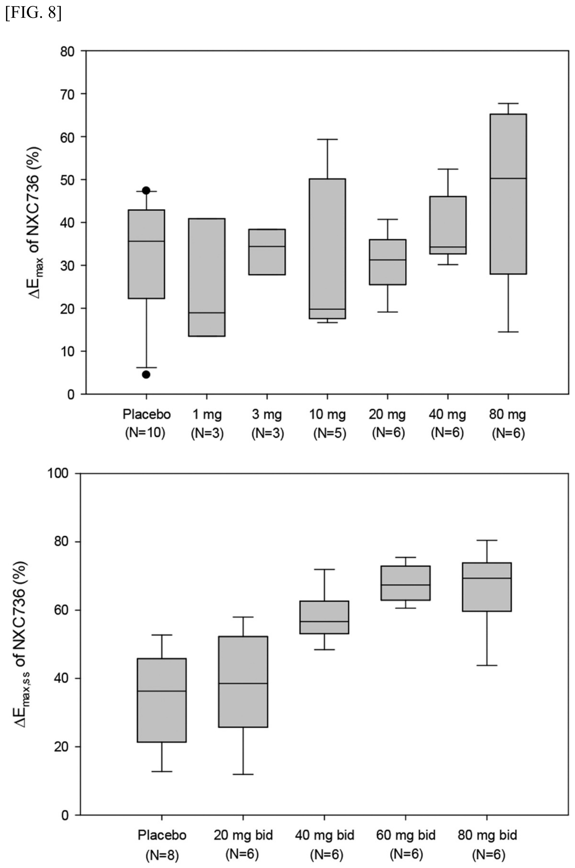

In addition, although the correlation between the systemic exposure and pharmacodynamic parameters of NXC736 was relatively unclear in the single-administration trial, it was confirmed in the repeated-administration trial that the degree of ALC reduction increased as the dose increased when 20 mg to 60 mg were administered twice daily, and a strong correlation was also observed with the systemic exposure of NXC736 (FIG. 8).

As a result, when NXC736 was administered twice a day at a dose of 40 mg or more, it showed a maximum ALC reduction effect greater than that of a single 80 mg dose, and the maximum ALC reduction when administered twice a day at a dose of 80 mg was similar to the maximum ALC reduction when administered twice a day at a dose of 60 mg, confirming that the pharmacological action reached a plateau when administered twice a day at a dose of 60 mg or more (FIG. 9).

Therefore, the effective dosage of NXC736 was determined to be 40 mg to 80 mg, which did not cause any side effects in phase 1 clinical trials, showed at least a 50% ALC reduction effect, and restored ALC to the original concentration when administration was discontinued, and the administration method was determined to be repeated administrations of twice a day.

## Claims

1. A pharmaceutical composition for preventing or treating a lymphocyte-mediated disease, comprising a compound represented by Chemical Formula 1 or a pharmaceutically acceptable salt, hydrate or solvate thereof as an active ingredient, wherein the active ingredient is administered to a subject in need thereof at a dose equivalent to 40 to 80 mg of the compound represented by Chemical Formula 1: 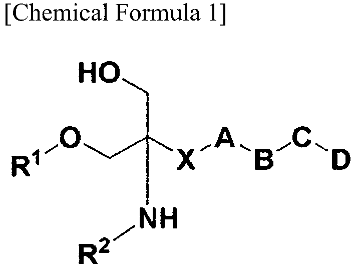
wherein R¹ is hydrogen;
R² is hydrogen or an acetyl group;
X is a single bond, C₂ alkylene or C₂ alkenylene;
A is a five-membered heteroarylene ring including three N atoms;
B is a C₂₋₁₁ straight-chain or branched-chain alkylene;
C is a single bond or phenylene; and
D is selected from the group consisting of hydrogen, phenyl and C₁₋₆ alkyl.

2. The pharmaceutical composition of claim 1, wherein the compound represented by Chemical Formula 1 is a compound represented by Chemical Formula 2:

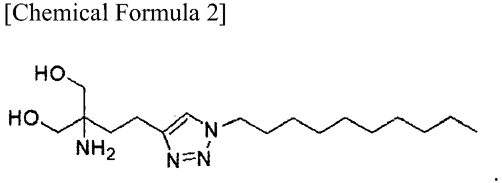

3. The pharmaceutical composition of claim 1, wherein the active ingredient is administered at a dose of 50 to 70 mg.

4. The pharmaceutical composition of claim 3, wherein the active ingredient is administered at a dose of 60 mg.

5. The pharmaceutical composition of claim 1, wherein the pharmaceutical composition is administered once a day or twice a day.

6. The pharmaceutical composition of claim 1, wherein the pharmaceutical composition is administered for at least 14 days.

7. The pharmaceutical composition of claim 1, wherein the pharmaceutical composition is administered orally.

8. The pharmaceutical composition of claim 1, wherein the pharmaceutical composition is formulated as a capsule or tablet that is suitable for oral administration.

9. The pharmaceutical composition of claim 1, wherein the pharmaceutical composition further comprises a pharmaceutically acceptable carrier.

10. The pharmaceutical composition of claim 1, wherein the pharmaceutical composition suppresses an absolute lymphocyte count to 50% or less.

11. The pharmaceutical composition of claim 1, wherein the lymphocyte-mediated disease is alopecia areata, inflammatory bowel disease, interstitial fibrosis and tubular atrophy or focal segmental glomerulosclerosis.
